# FASCICULE DE BREVET EUROPEEN

(11) **EP 0 508 914 B1**
(45) Date de publication et mention de la délivrance du brevet: **06.03.1996**
(21) Numéro de dépôt: 92420111.4
(22) Date de dépôt: 07.04.1992
(51) Int. Cl.: G01N 19/10, G01N 3/08, G01N 33/34, F16B 2/10, H01F 7/04, G01B 7/24

(54) **Appareil de mesure d'allongement de bandes échantillon**
Vorrichtung zur Messung der Dehnung von Bandproben
Apparatus for measuring the elongation of sample strips

(30) Priorité: 10.04.1991 FR 9104664
(43) Date de publication de la demande: 14.10.1992
(73) Titulaire: ASSOCIATION DE GESTION DE L'ECOLE FRANCAISE DE PAPETERIE ET DE L'IMPRIMERIE, F-75008 Paris (FR); Serra Tosio, Jean-Marie, F-38240 Meylan (FR); Chave, Yves, F-38610 Gieres (FR)
(72) Inventeur: Serra-Tosio, Jean-Marie, F-38240 Meylan (FR); Chave, Yves, F-38610 Gieres (FR)
(74) Mandataire: de Beaumont, Michel

(56) Documents cités:
- EP-A- 0 104 153
- DE-B- 2 943 691
- FR-A- 1 273 353

## Description

La présente invention concerne un appareil de mesure de l'allongement de bandes de papier placées dans une atmosphère contrôlée en humidité et en température utilisant de telles mâchoires.

L'une des caractéristiques d'un papier est sa dilatation en fonction de l'humidité de l'air, à température constante. Pour mesurer cette caractéristique, il existe des appareils dans lesquels on dispose une série de bandes échantillon de papier. Ces bandes échantillon sont soumises, dans une enceinte étanche, à des atmosphères d'humidité prédéterminées différentes et leurs variations de longueur sont mesurées. Les appareils classiques de mesure d'allongement des bandes comprennent, pour chaque bande, une mâchoire supérieure et une mâchoire inférieure dans lesquelles les extrémités de la bande sont maintenues. Chaque mâchoire supérieure ou inférieure est reliée à un instrument de mesure de déplacement et à un dispositif de réglage du zéro de l'instrument de mesure.

Dans un appareil du modèle "Neenah" disponible auprès de la société Technidyne, la mesure est faite par des micromètres reliés aux mâchoires supérieures et dont les molettes sont accessibles sur le dessus de l'appareil. Les mâchoires inférieures sont reliées à des leviers articulés dont l'horizontalité est contrôlée à vue par des niveaux à bulle. Initialement, la molette de chaque micromètre est actionnée pour déplacer verticalement la bande associée jusqu'à l'obtention de l'horizontalité du levier correspondant, ce qui se traduit par le centrage de la bulle du niveau associé. Pour mesurer les variations de longueur, la molette de chaque micromètre est à nouveau actionnée pour rendre les leviers horizontaux et l'allongement est alors déduit à partir de la rotation de la molette.

Dans un appareil du modèle 68936 disponible auprès de la société Sodexim S.A., la mesure de longueur est faite par des comparateurs mesurant la position des mâchoires inférieures. Initialement, chaque comparateur est mis à zéro en déplaçant verticalement la bande associée par l'intermédiaire d'une vis reliée à la mâchoire supérieure et accessible sur le dessus de l'appareil. Les allongements peuvent ensuite être directement lus sur les comparateurs.

Dans les appareils connus, il est donc nécessaire, pour chaque bande, d'ajuster le zéro d'un instrument de mesure associé et de relever la mesure, soit manuellement (micromètre), soit visuellement (comparateur), ce qui est fastidieux si le nombre de bandes est élevé. De plus, les mâchoires connues présentent divers inconvénients.

Un premier type connu de mâchoire est une pince à ressort. La pince à ressort est d'une manipulation facile, mais ne permet généralement pas de serrer uniformément les bandes de papier. Il en résulte que la bande peut se mettre de travers pendant ou après sa mise en place. Pour pallier cet inconvénient, le ressort des pinces peut être raidi, mais l'utilisation de ces pinces est alors plus désagréable, surtout si le nombre de pinces est élevé, car il faut fournir un effort important pour les ouvrir.

Un deuxième type connu de mâchoire plus fiable comprend un excentrique venant appuyer sur une lame en serrant la bande entre cette lame et un support. Toutefois, une telle mâchoire ne permet pas un bon serrage de bandes minces ou trop épaisses. De plus, l'effort de serrage à fournir est relativement important et le mouvement de serrage est peu commode, ce qui rend l'utilisation de ces mâchoires désagréable si leur nombre est élevé.

On connaît aussi des mâchoires magnétiques telles que décrites dans le document DE-B-29 43 691 utilisées pour fixer des feuilles de forme carrée sur un plan vertical.

Un objet de la présente invention est de prévoir un nouvel appareil de mesure d'allongement de bandes échantillon pour lequel l'insertion de bandes échantillon dans les mâchoires est particulièrement aisée.

Un autre objet de la présente invention est de prévoir un nouvel appareil de mesure d'allongement de bandes échantillon ne nécessitant pas plusieurs mises à zéro d'instruments de mesure.

Un autre objet de la présente invention est de prévoir un guidage de mâchoire inférieure permettant de simplifier davantage l'insertion des bandes échantillon.

La présente invention prévoit un appareil pour mesurer les allongements respectifs de plusieurs bandes échantillon, comprenant un socle, un châssis vertical et pour chaque bande : une mâchoire supérieure fixe pour serrer l'extrémité supérieure de la bande, une mâchoire inférieure mobile pour serrer l'extrémité inférieure de la bande et la tendre par gravité ; et un capteur de déplacement de la mâchoire inférieure, les mâchoires sont des mâchoires magnétiques dont chacune comprend une semelle et une plaque articulée sur la semelle, un aimant étant monté dans la semelle pour plaquer la plaque sur la semelle par attraction magnétique, et les semelles des mâchoires inférieures sont guidées verticalement par rapport au châssis.

Selon un mode de réalisation de la présente invention, la semelle de chaque mâchoire inférieure est en un matériau amagnétique et la mâchoire est guidée par une rainure réalisée dans une pièce en un matériau ferromagnétique solidaire du châssis, la mâchoire étant maintenue en guidage dans la rainure par la seule attraction de son aimant.

Selon un mode de réalisation de la présente invention, chaque mâchoire inférieure est reliée au capteur de déplacement associé par l'intermédiaire d'un dispositif d'accrochage magnétique.

Selon un mode de réalisation de la présente invention, chaque mâchoire comprend une articulation de la plaque sur la semelle présentant un jeu perpendiculaire à ladite face de contact.

Selon un mode de réalisation de la présente invention, la semelle de chaque mâchoire comprend des parois latérales servant de guide à la bande à serrer.

Selon un mode de réalisation de la présente invention, les capteurs de déplacement sont des capteurs à noyau plongeur et à transformateur différentiel, les noyaux plongeurs étant reliés aux mâchoires inférieures associées.

Selon un mode de réalisation de la présente invention, l'appareil comprend des moyens pour tendre davantage chacune des bandes par rajout de charges sur les mâchoires inférieures.

Selon un mode de réalisation de la présente invention, les capteurs de déplacement sont solidaires du socle et l'appareil comprend des moyens pour positionner le châssis à des positions haute et basse par rapport au socle.

Selon un mode de réalisation de la présente invention, l'appareil comprend une réglette horizontale disposée entre les mâchoires supérieures et inférieures, cette réglette ayant une position latérale où les mâchoires inférieures coulissent libredans le sens vertical et une autre position où les mâchoires inférieures butent contre la réglette.

Ces objets, caractéristiques et avantages ainsi que d'autres de la présente invention seront exposés plus en détail dans la description suivante de modes de réalisation particuliers faite en relation avec les figures jointes parmi lesquelles :
les figures 1A et 1B représentent respectivement une vue simplifiée en perspective et une vue de côté en coupe d'une mâchoire utilisée selon la présente invention ;
les figures 2A et 2B représentent respectivement une vue de côté en coupe et une vue de dessus d'un mode de réalisation particulier d'une mâchoire utilisée selon la présente invention ;
les figures 3A et 3B représentent respectivement une vue de face et une vue de côté d'un mode de réalisation d'un appareil de mesure d'allongement de bandes échantillon selon la présente invention ;
la figure 4 illustre une vue partielle de face du bâti de l'appareil des figures 3A et 3B ; et
la figure 5 illustre un mode d'accrochage d'une mâchoire inférieure à un capteur de déplacement et schématiquement un dispositif pour tendre davantage les bandes échantillon.

Dans les figures, des mêmes références désignent des mêmes éléments ou des éléments équivalents.

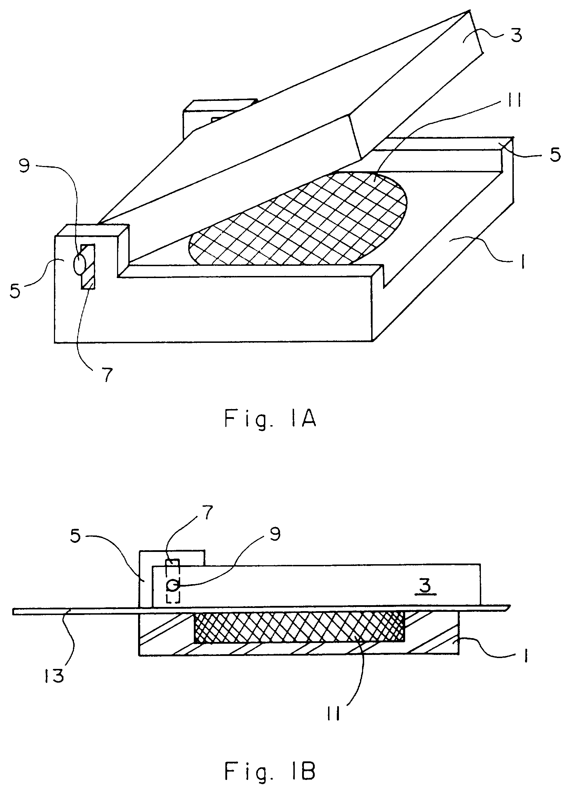

La figure 1A illustre une vue en perspective d'un mode de réalisation simplifié d'une mâchoire utilisée selon la présente invention. La mâchoire comprend une semelle 1 en un matériau amagnétique 1 sur laquelle est articulée une plaquette 3 venant en appui plan sur la surface supérieure de la semelle. La semelle 1 comprend des parois latérales ascendantes 5 qui maintiennent latéralement la plaquette 3. Les parois latérales 5 comprennent à l'avant des rainures verticales 7 dans lesquelles viennent se loger des axes 9 fixés latéralement à une extrémité de la plaquette 3. Un aimant permanent 11 est monté dans un logement de la semelle 1 et affleure la surface supérieure de la semelle. La plaquette 3 est en un matériau ferromagnétique et est donc susceptible de se coller sur l'aimant 11 en assurant un bon maintien d'un objet plat placé entre la plaquette 3 et la semelle 1.

La figure 1B illustre une vue de côté et en coupé de la mâchoire de la figure 1A en position fermée. On a représenté une bande de papier 13 serrée entre la plaquette 3 et la face supérieure de la semelle 1. Le positionnement latéral ou le guidage de la bande est assuré par les parois latérales 5.

On remarquera ici que la mâchoire est adaptée à recevoir des bandes de papier de différentes épaisseurs tout en maintenant un serrage plan des bandes. En effet, selon l'épaisseur de la bande, l'axe 9 se placera plus ou moins haut dans la rainure 7, la plaquette 3 restant parallèle à la surface supérieure de la semelle 1. Pour augmenter l'efficacité du maintien de la bande 13 lorsqu'elle est d'épaisseur importante, les surfaces de contact de la plaquette 3 et de la semelle 1 peuvent être striées transversalement, rendues rugueuses, ou recouvertes d'un matériau adhérent. On peut aussi munir l'une des faces de contact de picots venant dans des trous de l'autre face de contact.

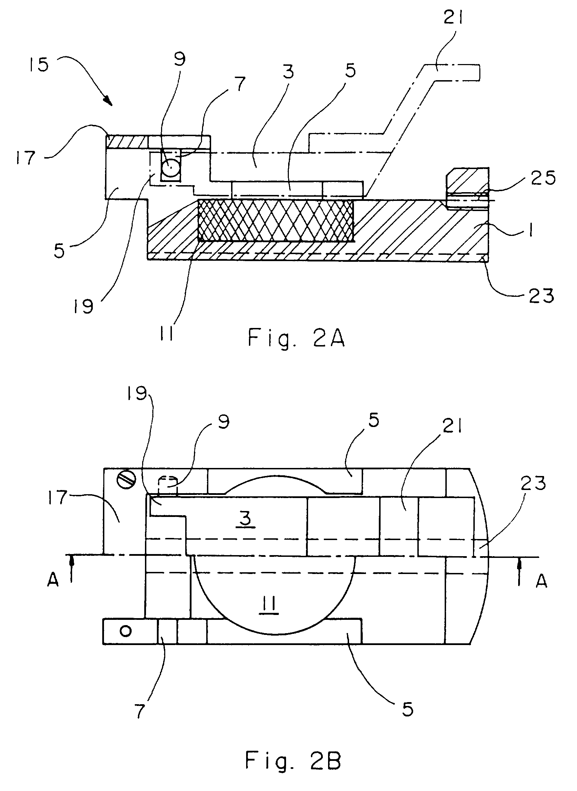

Les figures 2A et 2B représentent deux vues différentes d'un mode de réalisation particulier d'une mâchoire inférieure utilisée selon la présente invention comportant les mêmes éléments que celle des figures 1A et 1B. La figure 2A représente la mâchoire en vue de côté et en coupé selon un axe A-A. La figure 2B représente la mâchoire en deux demi-vues de dessus, une demi-vue représentant la mâchoire complète et l'autre demi-vue représentant la mâchoire nue. La plaquette 3 est représentée en traits mixtes à la figure 2A.

Les rainures 7 débouchent à la partie supérieure des parois latérales 5 et sont recouvertes par une plaque 17 vissée sur les parois latérales. La plaque 17 est échancrée, comme cela est représenté à la figure 2B, pour permettre à la plaquette 3 de se relever à une position approximativement perpendiculaire à la semelle 1. Les axes 9 sont emmanchés ou collés dans des oeillets latéraux 19 prolongeant longitudinalement la plaquette 3. Pour faciliter la manipulation de la mâchoire, la plaquette 3 est munie à l'arrière d'une languette ascendante 21.

La semelle 1 comporte à sa partie inférieure une nervure longitudinale 23 qui sert à guider la mâchoire, comme on le verra ultérieurement, dans une rainure d'une pièce en un matériau ferromagnétique. La mâchoire étant destinée à coulisser verticalement, il faut assurer le maintien de la nervure 23 dans la rainure. Pour cela, le fond de l'aimant 11 est suffisamment proche de la partie inférieure de la semelle 1 pour qu'il y ait une légère attraction magnétique entre l'aimant 11 et la pièce comprenant la rainure. On obtient ainsi un moyen particulièrement simple pour maintenir la mâchoire dans la rainure tout en permettant un glissement facile.

L'arrière de la semelle 1 est munie d'un épaississement ascendant comprenant un taraudage longitudinal 25 destiné, comme on le verra ultérieurement dans une application particulière, à relier la mâchoire à un capteur de déplacement.

De telles mâchoires sont particulièrement simples à manipuler. Elles assurent un positionnement et un guidage d'un objet en forme de bande. Une bande peut être introduite dans la mâchoire par les deux côtés. L'aimant permet d'assurer une double fonction, à savoir le serrage d'une bande et le maintien en position de la mâchoire contre un support en un matériau ferromagnétique.

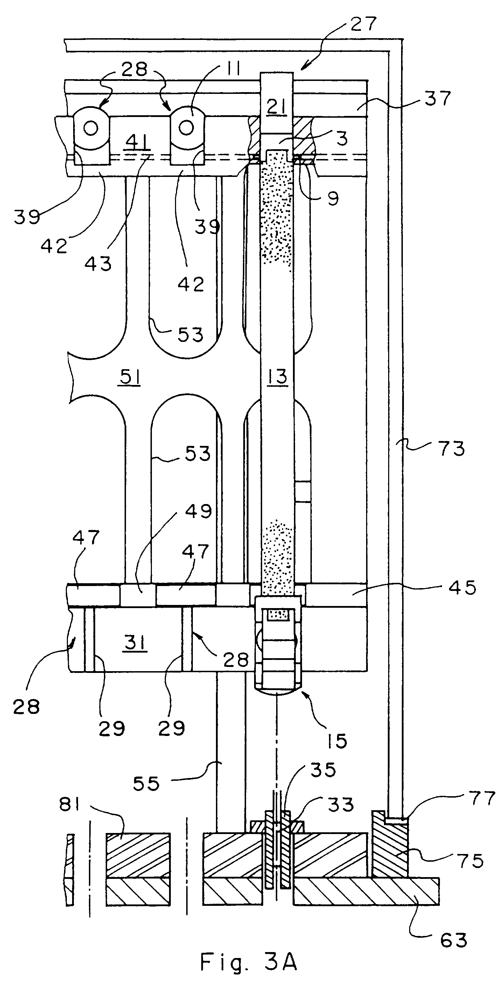

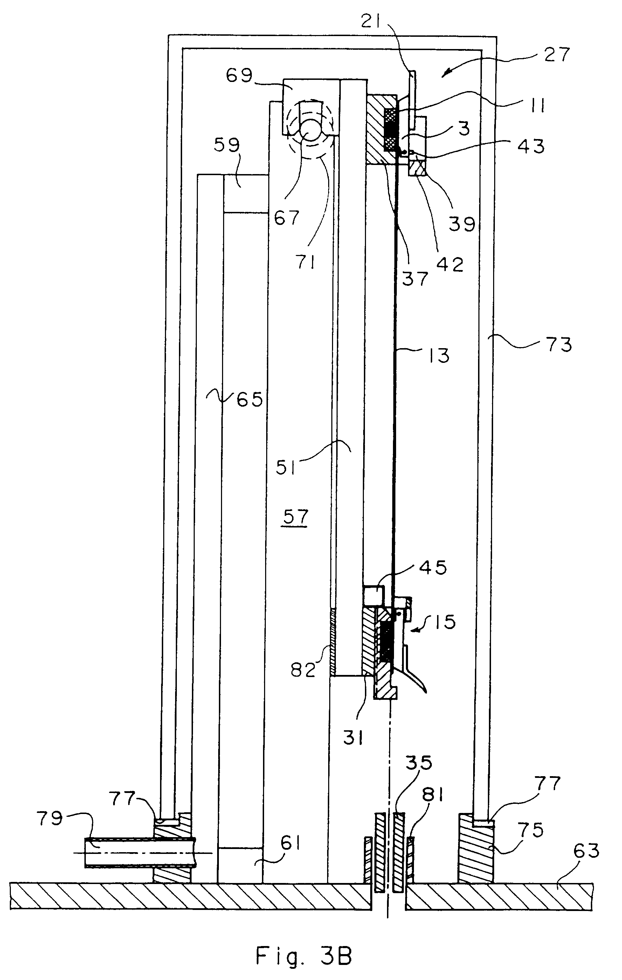

Les figures 3A et 3B représentent respectivement une vue de face et de côté d'un mode de réal isation d'un appareil selon la présente invention. Certains éléments ont été représentés en coupe ou en section pour mieux voir leur constitution. L'appareil est destiné à mesurer les allongements d'une série de bandes échantillon accrochées chacune verticalement entre une mâchoire supérieure et une mâchoire inférieure et soumises à une atmosphère conditionnée, telle que de l'air à taux d'humidité et température contrôlés. Cet appareil comprend, comme on le verra ci-après, divers aspects originaux et inventifs autres que l'utilisation des mâchoires décrites précédemment.

Dans les figures 3A et 3B, on a représenté une seule bande 13 maintenue à ses extrémités par une mâchoire inférieure 15 et une mâchoire supérieure 27. Quatre autres emplacements 28 de mâchoires supérieures et inférieures ont été représentés.

Les mâchoires inférieures 15 sont munies de nervures, telles que les nervures 23 précédemment décrites, qui coulissent dans des rainures verticales 29 d'une réglette horizontale inférieure fixe en un matériau ferromagnétique 31. Chaque mâchoire 15 est reliée à un élément mobile 33 d'un capteur de déplacement 35 par l'intermédiaire d'un dispositif d'accrochage magnétique décrit ultérieurement. De préférence, le capteur de déplacement 35 est un capteur électronique tel qu'un capteur à noyau plongeur 33 et à transformateur différentiel disponible auprès de la société Sensorex.

Les mâchoires supérieures 27 sont fixes et analogues aux mâchoires inférieures 15 sauf, comme cela sera éclairci ci-après, qu'elles ont une semelle commune. La semelle est remplacée par une réglette horizontale supérieure 37 dans laquelle sont montés longitudinalement des aimants 11 affleurant la face avant de la réglette. Les parois latérales de guidage 5 sont remplacées par des rainures verticales 39 d'une réglette horizontale 41 vissée sur la réglette 37. Les rainures 39 laissent apparents chacun des aimants 11 de la réglette 37 et chacune est arrêtée à son extrémité inférieure par une traverse 42 de la réglette 41. Comme cela est représenté à la figure 3B, cette traverse 42 ne ferme pas entièrement l'extrémité de la rainure 39 pour permettre le passage de la bande 13 vers le bas. Chaque rainure 39 reçoit une plaquette 3 de mâchoire, languette 21 vers le haut et axe d'articulation 9 vers le bas. Les axes d'articulation 9 sont logés dans une rainure horizontale 43 de la réglette 41, faisant face à la réglette 37 et débouchant dans les parois latérales des rainures 39 près des traverses 42.

La manipulation des mâchoires supérieures 27 est particulièrement pratique. En les ouvrant, les plaquettes 3 viennent reposer sur les traverses 42 et l'opérateur a les deux mains libres pour insérer la bande.

En contact avec la partie supérieure de la réglette 31 associée aux mâchoires inférieures, est disposée une réglette 45 qui coulisse latéralement. La réglette 45 comporte sur sa face avant des rainures verticales 47 plus larges que les mâchoires inférieures. Dans la position de la réglette 45 représentée à la figure 3A, les mâchoires inférieures 15 peuvent coulisser librement verticalement à travers les rainures 47. Cette réglette sert à ajuster la longueur initiale des bandes échantillon.

Le réglage de la longueur des bandes se fait à l'insertion de celles-ci. L'insertion et le réglage des bandes sont particulièrement commodes. On insère une extrémité d'une bande dans une mâchoire supérieure 27 ; on fait coulisser latéralement la réglette 45 de manière qu'une partie pleine 49 de celle-ci se trouve en face d'une rainure 29 ; On insère l'autre extrémité de la bande dans une mâchoire inférieure 15 en dessous de la réglette 45, la mâchoire inférieure viendra alors d'elle-même s'ajuster et se plaquer grâce à son aimant dans la rainure 29 ; on ouvre la mâchoire supérieure 27 et on abaisse la bande jusqu'à ce que la mâchoire inférieure 15 vienne s'accoupler à son capteur de déplacement par l'intermédiaire du dispositif d'accrochage magnétique décrit ultérieurement ; on tire la bande vers le haut jusqu'à ce que la mâchoire 15 vienne en butée contre la partie pleine 49 de la réglette 45 ; on ferme la mâchoire supérieure ; et on fait coulisser la réglette 45 dans sa position initiale représentée. Les mâchoires inférieures peuvent alors se déplacer librement vers le haut ou vers le bas.

Les réglettes 31, 37 et 45 sont montées sur un châssis 51. La réglette supérieure 37 peut être vissée sur le châssis 51 à plusieurs positions verticales, selon la longueur de bande souhaitée. Le châssis 51 est amovible, ce qui permet de prévoir un deuxième châssis identique que l'on pourra équiper d'avance d'une série de bandes échantillon pendant qu'on est en train d'effectuer des mesures avec le premier châssis. Pour une meilleure circulation de l'atmosphère au contact des bandes 13, des lumières 53 sont prévues dans le châssis à l'emplacement de chaque bande 13, mais le châssis peut être aussi constitué d'un cadre rigide.

Le châssis est monté sur un bâti dont est visible un montant latéral 55 à la figure 3A et un autre montant latéral 57 à la figure 3B. Les montants latéraux 55 et 57 sont vissés sur une entretoise supérieure 59 et sur une entretoise inférieure 61 elle-même vissée sur un socle horizontal 63. Pour assurer une meilleure rigidité du bâti, un montant 65 est vissé en déport vers l'arrière sur les entretoises 59 et 61. Les parties supérieures des nervures 55 et 57 sont traversées par un axe 67. Sur la partie supérieure du châssis 51 sont montées des chapes latérales 69, dont l'une est visible à la figure 3B, qui supportent le châssis 51 et reposent sur des excentriques 71 fixés sur les extrémités de l'axe 67. Les chapes sont prévues, comme cela est représenté, pour qu'on puisse désolidariser le châssis 51 du bâti en soulevant le châssis.

L'excentrique 71 visible à la figure 3B est représenté en pointillés dans ses positions extrêmes. Grâce aux excentriques 71, on peut mettre le châssis 51 à une position basse ou à une position haute pour des raisons que l'on verra plus loin.

Tous les éléments que l'on vient de décrire sont placés dans une enceinte étanche confinée à l'intérieur d'un capot 73, de préférence transparent, posé sur une embase 75 fixée sur le socle 63. Des joints d'étanchéité 77 sont prévus entre le capot et l'embase. L'embase 75 comprend à l'arrière deux embouts 79 d'arrivée et de sortie d'air conditionné ou autre gaz. Les capteurs de déplacement 35 sont montés dans une réglette 81 fixée sur le socle 63 et reliée à des circuits électroniques se trouvant sous le socle. Les montants 55 et 57 sont munis d'une plaque de frottement et de calage 82 aux endroits où la partie inférieure du châssis 51 appuie sur ces montants.

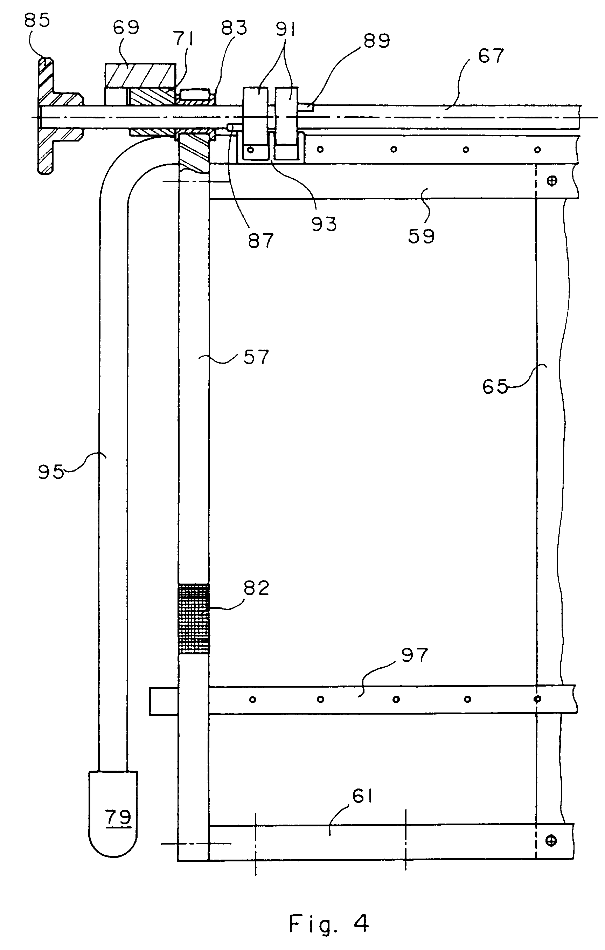

La figure 4 représente une vue partielle de face du bâti de l'appareil. L'axe 67 passe dans des paliers 83 des montants 55 et 57 et comprend à ses extrémités des molettes 85 permettant à un opérateur de positionner le châssis 51 à sa position basse normale ou à sa position haute. La position haute sert à étalonner les capteurs, l'étalonnage s'effectuant en déplaçant le châssis de sa position normale à la position haute et en vérifiant si les valeurs indiquées par un système d'acquisition de mesures relié aux circuits électroniques des capteurs correspondent au déplacement qui est connu avec précision. Si les valeurs indiquées ne correspondent pas, le système d'acquisition de mesures est réajusté.

Les positions haute et basse sont obtenues par les excentriques 71 qui appuient, comme cela est représenté, sur une partie horizontale des chapes 69. La rotation des excentriques 71 correspondant à ces deux positions est déterminée par deux butées 87 et 89 montées axialement dans des disques 91 solidaires de l'axe 67. Les butées 87 et 89 viennent en appui respectivement à l'avant et à l'arrière d'une pièce 93 montée sur l'entretoise supérieure 59.

Un embout 79 d'arrivée ou de sortie d'air conditionné est relié à un tube 95. Le tube 95 monte vers la partie supérieure de l'appareil où il s'étend horizontalement et où il comprend des orifices. Un tube 97 relié à l'autre embout (non représenté) comprend, au niveau de la partie inférieure de l'appareil, une partie horizontale percée.

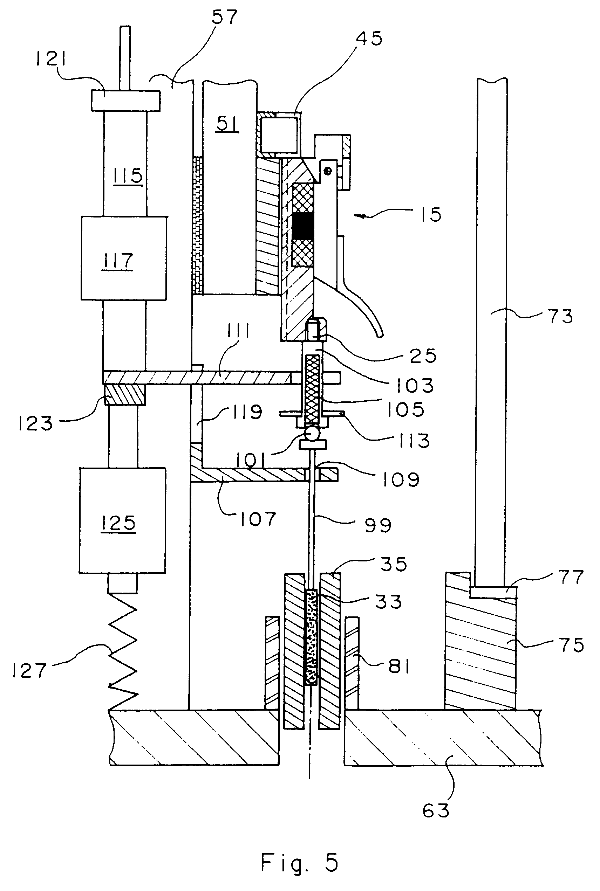

La figure 5 représente de façon plus détaillée le mode de fixation dune mâchoire inférieure 15 à son capteur de déplacement associé 35, et schématiquement un dispositif de lestage supplémentaire des bandes échantillon.

La fixation de la mâchoire au capteur se fait par un dispositif d'accrochage magnétique. A l'extrémité d'une tige 99 reliée au noyau plongeur 33 du capteur 35, est fixée une bille en matériau ferromagnétique 101. Un cylindre vertical creux en matériau amagnétique 103 est vissé dans le taraudage 25 de la mâchoire. Dans le cylindre 103 est logé un aimant 105 affleurant la partie inférieure du cylindre. Ainsi, quand on veut rendre solidaires le noyau plongeur 33 et la mâchoire 15, il suffit d'approcher le cylindre 103 de la bille 101 ou l'inverse et cette dernière vient se plaquer contre la partie inférieure du cylindre en se centrant dans la partie intérieure du cylindre.

Un tel dispositif d'accrochage présente plusieurs avantages. L'ensemble de la bille 101 plaquée sur son cylindre 103 est une liaison rotule qui assure un bon centrage du noyau plongeur 33 dans le capteur 35. L'attraction magnétique peut être suffisante pour que, lors de l'insertion, précédemment décrite, d'une bande, la mâchoire inférieure 15 vienne s'accoupler à son capteur sans qu'on le provoque délibérément au cours de la manipulation d'insertion. L'absence de liaison matérielle assure une manipulation simple et une protection des fragiles capteurs 35, notamment des tiges 99 et des noyaux plongeurs 33, dans le cas, par exemple, où on retire le châssis 51 sans préalablement désaccoupler les mâchoires 15 des capteurs.

Une équerre 107 fixée sur les nervures 55 et 57 comprend des trous verticaux 109 dans lesquels passent les tiges 99 des capteurs 35. Chacune des tiges 99 comprend à sa partie supérieure un épaulement qui empêche la tige de tomber à travers le trou 109 associé. Pour désaccoupler les mâchoires 15 des capteurs, on peut décoller les billes manuellement en tirant les tiges 99 vers le bas. On peut aussi incliner légèrement le châssis 51 autour de l'axe 67, les tiges 99 seront alors retenues dans les trous 109 et les billes 101 se décolleront par le mouvement du châssis.

L'appareil comprend aussi un dispositif de lestage supplémentaire des bandes échantillon servant à déterminer, par exemple, le module d'élasticité de chacune des bandes dans diverses conditions d'humidité. Ce dispositif comprend pour chaque bande, une fourche horizontale 111 qui, lorsqu'on veut charger davantage la bande, vient s'appuyer sur une rondelle 113 du cylindre 103. La fourche 111 est fixée à l'extrémité inférieure d'un axe vertical 115 coulissant dans une douille à billes 117 solidaire du bâti et servant de charge. La fourche 111 est guidée par une rainure verticale 119 de l'équerre 107. A la partie supérieure des axes 115, on peut rajouter des masses additionnelles 121. Les parties inférieures des axes 115 reposent sur une réglette horizontale 123 qui est fixée à la partie supérieure de pistons de deux vérins double effet 125 (un seul vérin est visible à la figure 5). Au repos, les pistons des vérins 125 sont maintenus dans une position haute où la fourche 111 n'appuie pas sur les rondelles 113, par deux ressorts 127 s'appuyant entre les parties inférieures des pistons des vérins et le socle 63. Lorsqu'on veut charger les bandes échantillon, les vérins 125 sont actionnés, les pistons de ceux-ci s'abaissent en écrasant les ressorts 127, les fourches 111 sont libérées et viennent appuyer sur les rondelles 113 en tendant davantage les bandes.

L'appareil selon la présente invention est prévu pour être utilisé avec une source d'air à taux d'humidité régulé électroniquement mis au point par le présent demandeur. Ainsi, l'ensemble de l'appareil selon la présente invention et de la source d'air peut être entièrement commandé par ordinateur, ce dernier fixant l'humidité dans l'enceinte par paliers maintenus pendant une durée suffisante, relevant les indications des capteurs de déplacement en déterminant les allongements et éventuellement actionnant les vérins 125 pour rajouter des charges supplémentaires aux bandes échantillon et déterminer le module d'élasticité des bandes d'après leurs allongements supplémentaires.

L'appareil de mesure d'allongement selon la présente invention est susceptible de nombreuses variantes et modifications qui apparaîtront à l'homme de l'art. L'appareil peut par exemple être muni de mâchoires classiques à pince ou à excentrique. Les divers éléments qui ne sont pas prévus pour être démontés peuvent être soudés au lieu d'être vissés. On peut utiliser l'appareil pour tout type de bande avec différents types d'atmosphère...

## Revendications

1. Appareil pour mesurer les allongements respectifs de plusieurs bandes échantillon (13), comprenant un socle (63), un châssis vertical (51) et pour chaque bande (13) :
- une mâchoire supérieure fixe (27) pour serrer l'extrémité supérieure de la bande (13) :
- une mâchoire inférieure mobile (15) pour serrer l'extrémité inférieure de la bande (13) et la tendre par gravité ; et
- un capteur de déplacement (33,35) de la mâchoire inférieure (15) ;
caractérisé en ce que les mâchoires (27,15) sont des mâchoires magnétiques dont chacune comprend une semelle (1) et une plaque (3) articulée sur la semelle (1), un aimant (11) étant monté dans la semelle (1) pour plaquer la plaque (3) sur la semelle (1) par attraction magnétique, et en ce que les semelles (1) des mâchoires inférieures (15) sont guidées verticalement par rapport au châssis (51).

2. Appareil selon la revendication 1, caractérisé en ce que la semelle (1) de chaque mâchoire inférieure (15) est en un matériau amagnétique et en ce que la mâchoire (15) est guidée par une rainure (29) réalisée dans une pièce en un matériau ferromagnétique solidaire du châssis (51), la mâchoire (15) étant maintenue en guidage dans la rainure (29) par la seule attraction de son aimant (11).

3. Appareil selon la revendication 1, caractérisé en ce que chaque mâchoire inférieure (15) est reliée au capteur de déplacement (33, 35) associé par l'intermédiaire d'un dispositif d'accrochage magnétique.

4. Appareil selon la revendication 1, caractérisée en ce que chaque mâchoire comprend une articulation (7, 9) de la plaque (3) sur la semelle (1) présentant un jeu perpendiculaire à ladite face de contact.

5. Appareil selon la revendication 1, caractérisée en ce que la semelle (1) de chaque mâchoire comprend des parois latérales (5) servant de guide à la bande à serrer.

6. Appareil selon l'une des revendications 1 ou 3, caractérisé en ce que les capteurs de déplacement (33,35) sont des capteurs à noyau plongeur (33) et à transformateur différentiel (35), les noyaux plongeurs (33) étant reliés aux mâchoires inférieures (15) associées.

7. Appareil selon la revendication 1, caractérisé en ce qu'il comprend des moyens pour tendre davantage chacune des bandes par rajout de charges sur les mâchoires inférieures (15).

8. Appareil selon la revendication 1, caractérisé en ce que les capteurs de déplacement (33,35) sont solidaires du socle (63) et en ce qu'il comprend des moyens (71) pour positionner le châssis (51) à des positions haute et basse par rapport au socle (63).

9. Appareil selon la revendication 1, caractérisé en ce qu'il comprend une réglette horizontale (45) disposée entre les mâchoires supérieures (27) et inférieures (15), cette réglette (45) ayant une position latérale où les mâchoires inférieures (15) coulissent librement dans le sens vertical et une autre position où les mâchoires inférieures (15) butent contre la réglette (45).

## Patentansprüche

1. Vorrichtung zum Messen der jeweiligen Ausdehnungen von mehreren Probenstreifen (13), die eine Basis (63) und einen vertikalen Rahmen (51) aufweist, und die für jeden Streifen (13) folgendes aufweist:
- eine fixierte obere Greifklaue (27), um die obere Extremität des Streifens (13) anzuziehen;
- eine bewegliche untere Greifklaue (15), um die untere Extremität des Streifens anzuziehen, und um diesen durch den Schwerkrafteffekt zu strecken; und
- einen Versetzungssensor (33, 35) für die untere Greifklaue (15);
dadurch **gekennzeichnet,** daß die Greifklauen (27, 15) magnetische Greifklauen sind, von denen jede eine Sohle (1) und eine Platte (3), die auf der Sohle (1) gelenkmäßig bzw. schwenkbar verbunden ist, aufweist, daß ein Magnet (11) in der Sohle (1) angebracht ist, um die Platte (3) gegen die Sohle (1) durch magnetische Anziehung zu drükken, und daß die Sohlen (1) der unteren Greifklauen (15) vertikal bezüglich des Rahmens (51) geführt sind.

2. Vorrichtung nach Anspruch 1, dadurch gekennzeichnet, daß die Sohle (1) jeder unteren Greifklaue (15) aus einem nicht-magnetischen Material hergestellt ist, und daß die Greifklaue (15) durch eine Nut (29) geführt ist, wobei die Nut (29) in einem Stück aus einem ferromagnetischen Material, das an dem Rahmen (51) fixiert ist, erreicht wird, und wobei die Greifklaue (15) in der Nut (29) gehalten und geführt wird, und zwar nur durch die Anziehung seines Magneten (11).

3. Vorrichtung nach Anspruch 1, dadurch gekennzeichnet, daß jede untere Greifklaue (15) mit dem entsprechenden Versetzungssensor (33, 35) durch magnetische Greifmittel gekoppelt ist.

4. Vorrichtung nach Anspruch 1, dadurch gekennzeichnet, daß jede Greifklaue eine Schwenkvorrichtung bzw. ein Gelenk (7, 9) der Platte (3) auf der Sohle (1) aufweist, und zwar mit einem Spiel senkrecht zu der Kontaktoberfläche.

5. Vorrichtung nach Anspruch 1, dadurch gekennzeichnet, daß die Sohle (1) jeder Greifklaue seitliche bzw. laterale Wände (5) zum Führen des anzuziehenden Streifen aufweist.

6. Vorrichtung nach Anspruch 1 oder 3, dadurch gekennzeichnet, daß die Versetzungssensoren (33, 35) Elektromagnetplungersensoren (33) einschließlich eines Differentialtransformators (35) sind und wobei die Elektromagnetplunger (33) mit den entsprechenden unteren Greifklauen (15) gekoppelt sind.

7. Vorrichtung nach Anspruch 1, dadurch gekennzeichnet, daß sie Mittel aufweist zum Erhöhen der Ausdehnung der Streifen durch Hinzufügen von Lasten auf den unteren Greifklauen (15).

8. Vorrichtung nach Anspruch 1, dadurch gekennzeichnet, daß die Versetzungssensoren (33, 35) an der Basis (63) fixiert sind, und dadurch, daß sie Mittel (71) aufweist zum Positionieren des Rahmens (51) in hohe und niedrige Positionen bezüglich der Basis (63).

9. Vorrichtung nach Anspruch 1, dadurch gekennzeichnet, daß sie eine horizontale Leiste bzw. Schiene oder Lineal (45) aufweist, und zwar angeordnet zwischen den oberen (27) und unteren (15) Greifklauen, wobei die Leiste (45) eine laterale Position besitzt, in der die unteren Greifklauen (15) frei entlang der vertikalen Richtung gleiten und eine weitere Position, in der die unteren Greifklauen (15) gegen die Leiste (45) anstoßen bzw. anliegen.

## Claims

1. An apparatus for measuring the respective elongations of several sample strips (13), comprising a base (63), a vertical frame (51), and for each strip (13):
- a fixed upper gripping jaw (27) to tighten the upper extremity of the strip (13);
- a mobile lower gripping jaw (15) to tighten the lower extremity of the strip and to stretch it by gravity effect; and
- a displacement sensor (33, 35) for the lower gripping jaw (15);
characterized in that the gripping jaws (27, 15) are magnetic gripping jaws, each of which comprises a sole (1) and a plate (3) articulated on the sole (1), a magnet (11) being mounted in the sole (1) to urge the plate (3) against the sole (1) by magnetic attraction, and that the soles (1) of the lower gripping jaws (15) are guided vertically with respect to the frame (51).

2. An apparatus according to claim 1, characterized in that the sole (1) of each lower gripping jaw (15) is made of a non-magnetic material and in that the gripping jaw (15) is guided by a groove (29) achieved in a piece made of a ferromagnetic material fixed to said frame (51), the gripping jaw (15) being maintained and guided in said groove (29) only by the attraction of its magnet (11).

3. An apparatus according to claim 1, characterized in that each lower gripping jaw (15) is coupled to the corresponding displacement sensor (33, 35) by a magnetic gripping means.

4. An apparatus according to claim 1, characterized in that each gripping jaw comprises an articulation (7, 9) of said plate (3) on the sole (1) having a clearance perpendicular to said contact surface.

5. An apparatus according to claim 1, characterized in that the sole (1) of each gripping jaw comprises lateral walls (5) for guiding the strip to be tightened.

6. An apparatus according to any of claim 1 or 3, characterized in that said displacement sensors (33, 35) are solenoid plunger sensors (33) including a differential transformer (35), said solenoid plungers (33) being coupled to said corresponding lower gripping jaws (15).

7. An apparatus according to claim 1, characterized in that it comprises means for increasing the elongation of said strips by addition of loads on said lower gripping jaws (15).

8. An apparatus according to claim 1, characterized in that said displacement sensors (33, 35) are fixed to said base (63), and in that it comprises means (71) for positioning said frame (51) to high and low positions with respect to said base (63).

9. An apparatus according to claim 1, characterized in that it comprises a horizontal ruler (45) disposed between said upper (27) and lower (15) gripping jaws, said ruler (45) having a lateral position where said lower gripping jaws (15) freely slide along the vertical direction and another position where said lower gripping jaws (15) abut against said ruler (45).
